(19) Europäisches Patentamt
European Patent Office
Office européen des brevets

(11) **EP 3 999 132 B1**

(12) **FASCICULE DE BREVET EUROPEEN**

(45) Date de publication et mention
de la délivrance du brevet:
**27.12.2023 Bulletin 2023/52**

(21) Numéro de dépôt: **20739724.1**

(22) Date de dépôt: **17.07.2020**

(51) Classification Internationale des Brevets (IPC):
*A61L 26/00* (2006.01)   *A61K 8/23* (2006.01)
*A61K 8/46* (2006.01)   *A61K 8/73* (2006.01)
*A61Q 17/00* (2006.01)

(52) Classification Coopérative des Brevets (CPC):
(C-Sets disponibles)
**A61L 26/008; A61L 26/0019; A61L 26/0023;**
A61L 2400/12                                    (Cont.)

(86) Numéro de dépôt international:
**PCT/EP2020/070323**

(87) Numéro de publication internationale:
**WO 2021/009360 (21.01.2021 Gazette 2021/03)**

(54) **TRAITEMENT ECOBIOLOGIQUE DES EFFETS SECONDAIRES DE LA RADIOTHERAPIE**

ÖKOBIOLOGISCHE BEHANDLUNG VON NEBENWIRKUNGEN DER RADIOTHERAPIE

ECOBIOLOGICAL TREATMENT OF SIDE EFFECTS OF RADIOTHERAPY

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **18.07.2019 FR 1908136**

(43) Date de publication de la demande:
**25.05.2022 Bulletin 2022/21**

(73) Titulaire: **Bionuclei**
**13290 Aix-en-Provence (FR)**

(72) Inventeurs:
• **ATHALIN, Han**
**44000 NANTES (FR)**
• **THOREL, Jean-Noël**
**75014 PARIS (FR)**

(74) Mandataire: **Cabinet Laurent & Charras**
**Le Contemporain**
**50 Chemin de la Bruyère**
**69574 Dardilly Cedex (FR)**

(56) Documents cités:
**EP-A1- 3 466 454    WO-A1-2019/229401**

EP 3 999 132 B1

**(Cont. page suivante)**

(52) Classification Coopérative des Brevets (CPC):
(Cont.)

C-Sets
**A61L 26/0019, C08L 81/00;**
**A61L 26/0023, C08L 5/04**

**Description**

DOMAINE DE L'INVENTION

La présente invention concerne une matrice bipolymérique tridimensionnelle déployant une activité biomécanique, apte à neutraliser les différents paramètres physiopathologiques impliqués dans le développement et l'aggravation des lésions et/ou plaies cutanées, notamment les radiodermites.

ETAT ANTERIEUR DE LA TECHNIQUE

Selon les dernières estimations publiées par le Centre International de Recherche sur le Cancer sur la base de données récoltées dans 185 pays, 18,1 millions de nouveaux cas de cancer auraient été diagnostiqués dans le monde en 2018.

En France, depuis 30 ans, le nombre global de nouveaux cas de cancer augmente chaque année. Cela s'explique principalement par le vieillissement de la population et l'amélioration des méthodes diagnostiques. Selon l'institut National du Cancer, en 2017, en France métropolitaine, on estime à 400 000 le nombre de nouveaux cas de cancers.

Il s'ensuit que le cancer demeure un problème de santé publique majeur en France et dans le monde.

En fonction de la nature de la tumeur, de son profil moléculaire et/ou de sa localisation, les traitements mis en oeuvre pour limiter ou stopper la progression de la maladie sont multiples. Qu'elles soient pratiquées seules ou en combinaison dans le cadre d'une prise en charge multidisciplinaire, on distingue classiquement la chirurgie, la radiothérapie, l'immunothérapie, l'hormonothérapie, les thérapies ciblées ou encore la chimiothérapie.

Ces traitements sont particulièrement agressifs pour l'organisme et s'accompagnent généralement d'effets secondaires.

En particulier, la radiothérapie (ou irradiation) consiste à utiliser des rayons à haute énergie pour détruire ou endommager les cellules cancéreuses. L'effet biologique principal des radiations ionisantes est de casser les brins d'ADN des cellules tumorales, soit directement, soit par le biais de la formation de radicaux libres, ce qui entraîne la mort de ces cellules.

Toutefois, une exposition à des radiations ionisantes induit l'apparition d'effets secondaires comme les radiodermites. Il s'agit de lésions cutanées dont la sévérité est classée en grade selon la classification internationale « Common Terminology Criteria for Adverse Events » :

- Grade 1 : érythème survenant en quelques jours jusqu'à trois semaines après la séance de radiothérapie, notamment chez les sujets à phototype de peau clair. Il est souvent accompagné de sensations de cuisson et d'oedème. Fréquent, il régresse rapidement à l'arrêt du traitement, après une phase de desquamation et souvent de dépilation transitoire par atteinte des annexes de la peau (follicules pileux, glandes sudoripares ou encore glandes sébacées).
- Grade 2 : érythème et oedème d'intensité moyenne, plaques exsudatives limitées au site d'irradiation.
- Grade 3 : radiodermite exsudative qui succède habituellement à l'érythème lorsque l'irradiation est poursuivie. Elle se manifeste par des décollements et des ulcérations confluentes laissant le derme à nu plusieurs semaines après l'irradiation. L'épithélialisation (ou reconstruction de l'épithélium) se fait en quelques semaines à plusieurs mois selon le siège de la lésion, avec pour conséquence une dyschromie et une alopécie le plus souvent définitives. Il faut habituellement interrompre la radiothérapie le temps de la cicatrisation.
- Grade 4 : radionécrose aiguë qui témoigne d'un surdosage, actuellement exceptionnelle sauf en cas de tumeurs très étendues en surface ou infiltrantes (irradiation massive en un temps très bref). Elle apparaît en quelques jours sous la forme d'un placard inflammatoire très douloureux avec phénomènes nécrotiques et hémorragiques évoluant vers une nécrose profonde, qui peut mettre à nu les muscles, les tendons et les os.

Pouvant donc aller du simple érythème sec jusqu'à la nécrose tissulaire, en passant par une phase intermédiaire plus ou moins exsudative, les radiodermites ne font l'objet d'aucun protocole de soins particulier. Aussi nombreux que variés, les traitements communément mis en oeuvre pour soigner ce type de lésions cutanées se rapprochent de ceux utilisés en cas de brûlure (émollients, dermocorticoïdes, pansements tels que des tulles gras, des hydrogels, des hydrocolloïdes ou encore des pansements au charbon ou à l'argent).

Le choix du meilleur traitement dépend directement du type de plaie à traiter, à savoir, sèche, suintante, fortement exsudative, infectée etc. Par exemple, un pansement de type hydrogel est de nature à favoriser la cicatrisation d'une lésion sèche, en la maintenant dans un environnement humide. En revanche, son application sur une radiodermite exsudative est susceptible de conduire à un phénomène de macération inhibant alors la cicatrisation et pouvant être à l'origine d'une infection ou surinfection.

De manière plus générale, la peau peut être lésée par d'autres moyens que le rayonnement ionisant de la radiothérapie.

Une plaie ou lésion cutanée est une interruption de la continuité des tissus. Il y a une plaie quand la peau ou la muqueuse est éraflée, coupée ou arrachée. La sévérité de la plaie est caractérisée par sa localisation, son aspect et son origine (brûlure, morsure, coupure etc).

Les lésions cutanées peuvent être classées en fonction de caractéristiques biologiques et cliniques telles que le besoin en hydratation/nutrition et le besoin en occlusion. Il est possible de distinguer 3 familles de lésions :

- Les lésions suintantes : il s'agit de lésions sujettes à macération qui nécessitent un assèchement avec un soin non-occlusif afin de laisser passer l'air. A titre d'exemple, on retrouve l'érythème fessier, la macération des plis, la varicelle avec lésions suintantes ou encore les ampoules.
- Les lésions non suintantes de type I : il s'agit de lésions superficielles à moyennes qui ont besoin d'hydratation avec un soin semi-occlusif, perspirant. A titre d'exemple, on retrouve les points de suture, les coupures, écorchures de la vie quotidienne après assèchement de la plaie, la varicelle en phase de cicatrisation, les lésions consécutives à un acte esthétique (peeling, laser, épilation définitive, tatouage, détatouage etc) ou encore l'érythème fessier non suintant.
- Les lésions non suintantes de type II : il s'agit de lésions moyennes à majeures qui exigent une nutrition relipidante avec un soin occlusif permettant de créer une barrière vis-à-vis de l'environnement extérieur. A titre d'exemple, on retrouve les gerçures, les dartres, les pulpites, les brûlures ou encore les excoriations.

La peau permet d'isoler et protéger l'organisme contre le milieu extérieur. Lorsqu'une plaie survient, le corps enclenche un phénomène biologique naturel : la cicatrisation.

Elle représente un processus de réparation complexe au cours duquel l'organisme doit arrêter une éventuelle hémorragie, protéger, assainir et refermer la plaie. Le tissu lésé doit se reconstituer, le plus proche possible du tissu initial.

Le processus naturel de cicatrisation d'une plaie correspond à une succession d'étapes biochimiques, biologiques et cellulaires, toutes indispensables à la guérison.

La cicatrisation peut se définir comme un phénomène comprenant 3 phases successives, caractérisées par des activités cellulaires spécifiques, qui font progresser le processus de réparation selon des séquences chronologiques précises et imbriquées les unes aux autres :

Étape 1 - phase inflammatoire ou de détersion : Tout de suite après la constitution de la plaie, les vaisseaux sanguins locaux se dilatent provoquant une augmentation de la perméabilité vasculaire et une fuite de plasma. Cette vasodilatation est suivie peu après d'une vasoconstriction puis de la formation de caillots au fond de la plaie grâce notamment à l'action des plaquettes, ce qui limite la perte de sang. Plus tard, attirés par des substances chimiotactiques, des cellules pro-inflammatoires (leucocytes et macrophages) arrivent des tissus avoisinants pour nettoyer la plaie, éliminant les tissus morts, germes et bactéries. Cette phase débute entre la 12$^{ème}$ et la 24$^{ème}$ heure et provoque une réaction inflammatoire caractérisée par une rougeur (érythème), un gonflement (oedème), une douleur et une augmentation de la température locale.

Bien que nécessaire, il est important que cet état inflammatoire ne dure pas plus de trois jours, sous peine de retarder les étapes suivantes de prolifération cellulaire et de formation d'un nouvel épithélium ou (épithélialisation). Or, dans certains cas, l'inflammation peut être stimulée et prolongée par la présence de radicaux libres comme les espèces réactives de l'azote (ou ERN ou RNS) et les espèces réactives de l'oxygène (ERO ou ROS). Ces composés vont naturellement induire une oxydation des biomolécules nouvellement synthétisées dans la plaie et donc ralentir le processus de régénération cellulaire et de cicatrisation.

Étape 2 : Phase de bourgeonnement ou de formation de tissu de granulation : Pendant cette phase, des cellules du tissu conjonctif, les fibroblastes, apparaissent en grande quantité après stimulation et recrutement par les macrophages. Les fibroblastes produisent des quantités importantes de collagène, d'élastine et autres éléments de la matrice cellulaire du derme. En même temps, les cellules endothéliales forment des bourgeons aux extrémités des capillaires lésés. Cette prolifération s'arrête lorsque le tissu de granulation a comblé la perte de substance et que les fibroblastes ont atteint les berges de la plaie.

Étape 3 : phase d'épidermisation ou d'épithélialisation : Une fois la cicatrice primaire formée, autour du 25$^{ème}$ ou 30$^{ème}$ jour, le collagène commence à se dégrader de façon importante et marque le début du remodelage de la cicatrice primaire. Au cours de cette phase, les berges de la plaie continuent à se contracter lentement grâce à l'action des myofibroblastes et au renforcement de l'union entre l'épiderme et le derme. Le résultat est que peu à peu, la cicatrice devient plus souple, plus lisse et plus douce au toucher. Ce remodelage aboutit à la formation de la cicatrice définitive au bout de 6 mois à un an voire plus.

La qualité de la cicatrice définitive dépend de sa taille, de sa situation et tout particulièrement de l'évolution initiale de la cicatrisation, d'où l'importance des soins et du suivi lors de la formation de la cicatrice primaire.

Une altération du processus de cicatrisation primaire d'une plaie aiguë peut conduire à la formation d'une plaie chronique et compliquée nécessitant des soins spécialisés avec des pansements et du matériel permettant une cicatrisation dite "dirigée", ou à la constitution d'une cicatrice anormale, peu esthétique et pouvant même présenter des séquelles fonctionnelles.

Une plaie qui n'est pas traitée correctement présente un risque d'infection important, ayant alors des conséquences sur le processus de cicatrisation. On parle de « contamination » lorsque le tissu endommagé est peuplé de bactéries, qui ne se multiplient pas encore. En revanche, la phase de « colonisation » implique une prolifération bactérienne au sein de la plaie. La colonisation peut se transformer en « colonisation critique » puis en infection locale de la plaie. Les bactéries ont alors pénétré plus profondément dans le tissu lésé et s'y multiplient en provoquant des réactions inflammatoires. Les symptômes généraux d'une telle infection sont la fièvre, l'apparition de rougeurs, une douleur au niveau de la plaie ou encore une leucocytose. Par le biais de la circulation sanguine, l'infection locale peut se transformer en « infection systémique » et se répandre dans l'ensemble du corps, voire se transformer en septicémie aigüe pouvant entraîner la mort du sujet.

Dans le domaine des soins des plaies, le choix du pansement contribue de manière décisive à garantir son efficacité thérapeutique et à réaliser le soin dans les conditions d'asepsies requises.

Il apparaît clairement que le choix du meilleur traitement dépend directement du type de plaie à traiter. Pour atteindre une efficacité maximum un pansement devrait idéalement combiner de nombreuses caractéristiques et fonctionnalités telles qu'être capable de prévenir les risques d'infection, d'absorber les excès d'exsudats et de sang, de créer ou maintenir un environnement humide autour de la plaie, faciliter la migration des leucocytes, maintenir une bonne isolation thermique ou encore être respirant.

Aujourd'hui un tel spectre de fonctionnalités n'est pas réuni au sein d'un même pansement. Il est nécessaire d'adapter le pansement au type de lésion, voire de le faire évoluer au fur et à mesure des étapes de cicatrisation.

Le document EP 3 466 454 décrit une matrice bipolymérique comprenant un polymère sulfaté. La matrice est obtenue par la formation d'un hydrogel, produit de la réaction entre les deux polymères.

Il subsiste donc un besoin évident de mettre au point un pansement unique capable de soigner avec un maximum d'efficacité tous les types de lésions cutanées et/ou les grades de sévérité d'une plaie, notamment la radiodermite.

## EXPOSE DE L'INVENTION

Le Demandeur a constaté qu'une matrice bipolymérique tridimensionnelle permet d'organiser et contrôler les conditions indispensables à la cicatrisation d'une plaie (humidité, température, pH, oxygénation), quel que soit le type de plaie, son degré d'évolution et/ou son degré de cicatrisation.

La présente invention permet de résoudre les problèmes de l'art antérieur évoqués précédemment.

Selon un premier aspect, l'invention concerne une matrice bipolymérique tridimensionnelle déployant une activité biomécanique, apte à neutraliser les différents paramètres physiopathologiques impliqués dans le développement et l'aggravation des lésions et/ou plaies cutanées combinant :

- un premier réseau polymérique comprenant des premiers colloïdes (Col-1) liés de manière non covalente avec un polysaccharide réticulé non sulfaté; et
- un second réseau polymérique réticulé comprenant des seconds colloïdes (Col-2) liés de manière covalente ou non covalente avec un polysaccharide sulfaté.

Au sens de l'invention, par « colloïde », on désigne des particules cristallines (forme non amorphe) résultant de l'empilement ordonné de molécules les constituant. Ces colloïdes peuvent également être appelés « quantum dots » (points quantiques) ou nanocristaux. Ils peuvent se présenter sous la forme d'une suspension de colloïdes dans un milieu aqueux.

La notion de liaison des colloïdes Col-1 et Col-2 fait partie des connaissances générales de l'homme du métier. La liaison correspond à la formation de liaisons non covalentes ou de liaisons covalentes.

Bien entendu, la liaison non covalente ou covalente ne se limite pas à la liaison d'un seul composé. Il s'agit éventuellement de la liaison d'une multitude de molécules d'au moins un type de composé sur chaque nanocristal.

La formation du complexe Col-2/polysaccharide sulfaté est réalisée selon les connaissances de l'homme du métier, notamment la formation de liaison covalente ou non covalente.

Dans un mode de réalisation particulier, les seconds colloïdes (Col-2) sont liés de manière covalente avec le polysaccharide sulfaté.

A titre d'exemple, le polysaccharide sulfaté peut être modifié par ajout de groupements thiols (R-S-H). Lorsque les colloïdes Col-2 sont placés en présence du polysaccharide sulfaté modifié par ajout de groupements thiols, des liaisons covalentes sont formées entre les atomes de soufre (S) des groupements thiols portés par le polysaccharide sulfaté et les molécules de métal constitutifs des colloïdes Col-2.

Dans un mode de réalisation privilégié, les seconds colloïdes (Col-2) sont liés de manière non-covalente avec le polysaccharide sulfaté.

A titre d'exemple, le colloïdes Col-2 peuvent être liés en surface de manière covalente avec un agent apte à charger positivement le colloïde, avantageusement la cystéamine, ce qui permet d'instaurer des interactions électrostatiques non covalentes entre les seconds colloïdes (Col-2) et le polysaccharide sulfaté, chargé négativement.

Au sens de l'invention, par « chargé positivement » et « chargé négativement » on désigne la charge présentée à la surface des colloïdes à pH neutre, physiologique ou acide (pH 3-7).

Les colloïdes peuvent être synthétisés selon les techniques conventionnelles, par exemple par l'approche dite « bottom-up » de croissance de précurseurs. Cette voie de synthèse, couramment utilisée dans le domaine des nanomatériaux, met en oeuvre une étape de nucléation et une étape de croissance à partir d'atomes isolés. Elle permet de contrôler la taille des colloïdes.

Les colloïdes Col-1 et Col-2 sont différents l'un de l'autre, c'est-à-dire constitués d'au moins un élément chimique différent. En effet, ils ne présentent pas les mêmes propriétés.

Selon un mode de réalisation particulier, les colloïdes Col-1 sont constitués d'un élément chimique choisi dans le groupe comprenant Ce, Si, Ge, Sn, Te, B, N, P, As, Al, Sb, Ga, In, Cd, Zn, Cu, Cl, Pb, Tl, Bi, Ti, U, Ba, Sr, Li, Nb, La, I, Mo, Mn, Ca, Fe, Ni, Eu, Cr, Br, Ag, Pt, Hg, et leurs assemblages.

Selon un mode de réalisation particulier, les colloïdes Col-2 sont constitués d'un élément chimique, de préférence un métal, avantageusement choisi dans le groupe comprenant Pt, Au, Ni, Cu, Pd et Ag.

Selon un mode de réalisation particulier, le métal constituant les colloïdes Col-2 est de degré d'oxydation zéro.

Selon un mode de réalisation particulier, les colloïdes Col-1 selon l'invention sont des colloïdes de dioxyde de cérium ($CeO_2$).

Selon un mode de réalisation particulier, les colloïdes Col-2 selon l'invention sont des colloïdes de platine (Pt).

Avantageusement, les colloïdes Col-2 présente des groupement amines à leur surface.

Au sens de l'invention, les colloïdes Col-2 ont un coeur constitué d'un métal, alors qu'ils ont une surface liée de manière covalente ou non covalente avec un polysaccharide sulfaté. Il s'agit d'une notion de type coeur/surface. Le terme de « coeur » n'est pas à associer à une structure de type coeur/coquille.

La surface des colloïdes Col-2 peut éventuellement présenter une couche d'oxyde. Dans ce cas, les colloïdes présentent un coeur en métal et une surface en oxyde, laquelle est liée de manière covalente avec un polysaccharide sulfaté.

Avantageusement, les colloïdes Col-1 sont des colloïdes de dioxyde de cérium ($CeO_2$) et les colloïdes Col-2 sont des colloïdes de platine (Pt) de degré d'oxydation zéro, avantageusement de platine dont le coeur est de degré d'oxydation zéro.

De manière générale, les colloïdes Col-1 et/ou les colloïdes Col-2 présentent une taille moyenne de l'ordre de quelques nanomètres à quelques dizaines de nanomètres.

Ainsi, les colloïdes Col-1 et/ou les colloïdes Col-2 selon l'invention présentent une taille avantageusement comprise entre 0,1 nm et 1000 nm, plus avantageusement entre 0,3 nm et 100 nm, et encore plus avantageusement inférieur à 10 nm, voire inférieure à 5 nm, la taille étant avantageusement mesurée par DRX.

Avantageusement, les colloïdes Col-1 présentent une taille comprise entre 0,4 nm et 2 nm.

Avantageusement, les colloïdes Col-2 présentent une taille comprise entre 1 et 5 nm, avantageusement entre 2 et 3 nm.

La technique DRX (diffraction des rayons X) est une technique conventionnellement utilisée pour mesurer la taille de cristaux à l'état solide.

Par taille, on désigne la dimension la plus importante des colloïdes Col-1 et Col-2, par exemple le diamètre dans le cas de colloïdes Col-1 et Col-2 de forme sphérique. Il s'agit de la taille moyenne en nombre des colloïdes Col-1 liés de manière non covalente et des colloïdes Col-2 liés de manière covalente. Néanmoins, la taille des colloïdes préalablement à la liaison non covalente ou covalente, éventuellement enrobés d'un polymère tel que le dextrose, le myo-inositol ou la polyvinylpyrrolidone (PVP), est également comprise dans les plages de valeurs indiquées ci-dessus. Le cas échéant, l'homme du métier saura adapter la taille des colloïdes de $CeO_2$ non liés et des colloïdes de Pt non liés.

L'enrobage des colloïdes selon l'invention, permet de contrôler leur croissance lors de leur formation. A titre d'exemple, dans un complexe Col-1/dextrose ou col-1/myo-inositol, le dextrose et le myo-inositol assurent un rôle de contrôle de la taille des particules de Col-1 pendant leur formation.

Au sens de l'invention, par « contrôle de la croissance des colloïdes », on désigne le mécanisme par lequel la taille des colloïdes est moins élevée et moins polydispersée, c'est-à-dire que toutes les particules ont des tailles suffisamment proches en formant une distribution étroite autour de la moyenne.

Les colloïdes selon l'invention sont avantageusement de forme sphérique.

Les colloïdes ne sont pas dopés. Eventuellement, ils peuvent comprendre un élément, de préférence un métal de transition, qui est introduit lors de la synthèse des colloïdes.

Selon un mode de réalisation particulier, le polysaccharide non sulfaté selon l'invention a une masse moléculaire comprise entre 1 kDa et 5 millions de Dalton (MDa), avantageusement entre 5 kDa et 1 MDa.

Selon un mode de réalisation particulier, le polysaccharide non sulfaté selon l'invention est choisi dans le groupe comprenant : l'alginate, l'acide hyaluronique, la gomme guar, la gomme xanthane, la gomme acacia, le pullulan, le dextrane et leurs mélanges, avantageusement l'alginate.

Avantageusement, le premier réseau polymérique selon l'invention est réticulé par un agent réticulant correspondant avantageusement à un métal, de préférence un métal bivalent, encore plus avantageusement un métal alcalinoterreux, par exemple le calcium ou le magnésium. Cet agent réticulant, notamment le calcium.

De préférence, le réseau polymérique selon l'invention correspond à de l'alginate réticulé au moyen d'un métal.

Selon un mode de réalisation particulier, le ratio massique agent réticulant/polysaccharide non sulfaté est compris entre 1 et 10, avantageusement entre 1 et 4.

Selon un mode de réalisation particulier, le ratio massique colloïdes Col-1 /polysaccharide non sulfaté est compris entre 1/50 et 1/1, avantageusement entre 1/15 et 1/5, de préférence le ratio massique est de 1/10.

Selon un mode de réalisation, dans le complexe colloïdes Col-1 /polysaccharide non sulfaté selon l'invention, les colloïdes Col-1 sont des colloïdes de dioxyde de cérium et le polysaccharide non sulfaté est choisi dans le groupe comprenant : l'alginate, l'acide hyaluronique, la gomme guar, la gomme xanthane, la gomme acacia, le pullulan, le dextrane et leurs mélanges, avantageusement l'alginate.

Selon un mode de réalisation particulier, le polysaccharide sulfaté selon l'invention a une masse moléculaire comprise entre 1 et 40 millions de Dalton (MDa), avantageusement entre 5 et 30 MDa, de préférence entre 15 et 25 MDa.

Selon un mode de réalisation particulier, le polysaccharide sulfaté selon l'invention est choisi dans le groupe comprenant le composé dont la désignation INCI est Aphanothece Sacrum Polysaccharides ; les glycosaminoglycanes sulfatés, par exemple la dermatane sulfate, l'héparine, le sulfate d'héparane ou encore la chondroïtine sulfate ; les glucanes ; les fucanes ; les fucoïdanes ; les carraghénanes ; les ulvanes, le pentosane polysulfate et leurs mélanges, avantageusement le Aphanothece Sacrum Polysaccharides.

Dans un mode de réalisation particulier, le polysaccharide sulfaté selon l'invention est l'Aphanothece Sacrum Polysaccharides (INCI) modifié par ajout de groupements thiols, avant sa mise en contact avec les colloïdes Col-2.

Avantageusement, le second réseau polymérique selon l'invention est réticulé par les colloïdes Col-2, avantageusement des colloïdes de platine de degré d'oxydation zéro, liés en surface et de manière covalente avec le composé correspondant à la désignation INCI Aphanothece Sacrum Polysaccharides modifié par ajout de groupements thiols.

Dans un autre mode de réalisation particulier, le colloïdes Col-2, avantageusement des colloïdes de platine de degré d'oxydation zéro, sont liés en surface de manière covalente avec un agent apte à charger positivement le colloïde, avantageusement la cystéamine, ce qui permet d'instaurer des interactions non covalentes entres les groupement amines de Col-2 et les sulfates des polysaccharides sulfatés selon l'invention, avantageusement le Aphanothece Sacrum Polysaccharides (INCI).

Dans les deux modes de réalisation décrits précédemment, les colloïdes Col-2 peuvent agir en tant qu'agent réticulant.

Selon un mode de réalisation particulier, le ratio massique colloïdes Col-2/polysaccharide sulfaté est compris entre 1/50 et 1/1, avantageusement entre 1/25 et 1/15, de préférence le ratio massique est de 1/20.

Selon un mode de réalisation particulier, le ratio colloïdes Col-1 liés de manière non-covalente /colloïdes Col-2 liés de manière covalente ou non covalente est compris entre 30/1 et 2/1, avantageusement entre 15/1 et 5/1.

Selon un mode de réalisation, dans le complexe colloïdes Col-2 /polysaccharide sulfaté selon l'invention, les colloïdes Col-2 sont des colloïdes de platine et le polysaccharide sulfaté est choisi dans le groupe comprenant le composé dont la désignation INCI est Aphanothece Sacrum Polysaccharides ; les glycosaminoglycanes sulfatés, par exemple la dermatane sulfate, l'héparine, le sulfate d'héparane ou encore la chondroïtine sulfate ; les glucanes, les fucanes, les fucoïdanes, les carraghénanes, les ulvanes, le pentosane polysulfate et leurs mélanges, avantageusement l'Aphanothece Sacrum Polysaccharides

Selon un mode de réalisation particulier, la matrice bipolymérique tridimensionnelle selon l'invention comprend de l'eau.

Selon un autre mode de réalisation particulier, les colloïdes Col-1 liés de manière non covalente avec un polysaccharide non sulfaté représentent entre 0,01% et 1% en poids de la matrice bipolymérique selon l'invention, tandis que les colloïdes Col-2 liés de manière covalente ou non covalente avec un polysaccharide sulfaté représentent entre 0,001% et 0, 1% et l'eau représente entre 70% et 99%.

Selon un mode de réalisation particulier, la matrice bipolymérique selon l'invention contient :

- des colloïdes de dioxyde de cérium ;
- de l'alginate ;
- du carbonate de calcium ;
- des colloïdes de platine de degré d'oxydation zéro, avantageusement de platine dont le coeur est de degré d'oxydation zéro ;
- de la polyvinylpyrrolidone ;
- de l'acide gluconique ou de la gluconolactone, avantageusement de la gluconolactone ;

- de l'Aphanothece Sacrum Polysaccharides (INCI) modifié par ajout de groupements thiols ; et
- de l'eau.

Selon un mode de réalisation préféré la matrice bipolymérique selon l'invention contient

- des colloïdes de dioxyde de cérium ;
- de l'alginate ;
- du carbonate de calcium ;
- des colloïdes de platine de degré d'oxydation zéro, avantageusement de platine dont le coeur est de degré d'oxydation zéro, modifié par ajout de cystéamine ;
- de la gluconolactone ;
- de l'Aphanothece Sacrum Polysaccharides (INCI) et
- de l'eau.

Selon un autre aspect, l'invention concerne une matrice telle que définie précédemment pour utilisation comme pansement pour prévenir et/ou cicatriser une plaie cutanée.

Selon un autre mode de réalisation, la plaie cutanée est sèche ou exsudative.

Selon un autre aspect, l'invention concerne une matrice telle que définie précédemment pour utilisation comme pansement pour prévenir et/ou cicatriser une radiodermite.

Selon un autre mode de réalisation, la radiodermite est de grade sec à exsudatif.

En d'autres termes, il s'agit des radiodermites de grade 1, 2 ou 3.

En particulier, la matrice bipolymérique selon l'invention correspond à un enchevêtrement de deux réseaux réticulés créant autour de la plaie un environnement favorable à la prévention de l'aggravation de ladite plaie et/ou à une accélération de sa guérison (humidité, température, pH, oxygénation, fonctions antibactériennes et fonctions antioxydantes).

Avantageusement, la matrice bipolymérique selon l'invention correspond à une matrice réticulée par des liaisons ioniques.

En cas de plaie, la matrice bipolymérique selon l'invention pour son utilisation comme pansement permet d'assurer un environnement humide favorable à la rapidité et à la qualité de la cicatrisation. En particulier, cet environnement humide est propice à la division et à la migration des cellules nouvellement créées au cours de la phase d'épithélialisation. L'humidification de la plaie empêche également la formation d'une croûte de surface ralentissant la cicatrisation complète et affranchit le patient d'avoir à en subir certains effets secondaires délétères tels qu'une légère dépression à l'endroit de la lésion.

De plus, la matrice bipolymérique pour son utilisation selon l'invention permet de maintenir la plaie à une température proche de la température corporelle, soit environ 37°C, ce qui assure une bonne prolifération des kératinocytes et des conditions optimales pour la réalisation des réactions enzymatiques.

La matrice bipolymérique pour son utilisation selon l'invention assure le maintien d'un pH entre 7 et 7,6 assurant une prolifération épithéliale tout en limitant la prolifération des organismes pathogènes dans la plaie.

La matrice bipolymérique pour son utilisation selon l'invention assure le rôle de barrière physique, naturellement dévolue à la peau, mais que cette dernière ne peut plus remplir après avoir été lésée. Le pansement selon l'invention assure une oxygénation, c'est-à-dire les échanges gazeux, entre la plaie et l'environnement extérieur, indispensables à la cicatrisation.

La matrice bipolymérique pour son utilisation selon l'invention possède un pouvoir absorbant efficace, important, élevé, des saignements et exsudats sécrétés par la plaie. En conséquence, le pansement selon l'invention n'a pas à être changé fréquemment et n'induit pas un bouleversement du processus de reconstruction de l'épiderme.

Selon l'invention, l'alginate et le composé correspondant à la désignation INCI Aphanothece Sacrum Polysaccharides permettent une absorption des fluides et exsudats ; participent à la résistance mécanique et à l'élasticité et jouent un rôle d'isolant via un maintien de la température et du pH de la plaie.

En parallèle, le complexe formé par les colloïdes Col-1 de $CeO_2$ liés de manière non covalente avec l'alginate a un rôle antioxydant permettant de contrôler l'inflammation sans l'inhiber totalement. En d'autres termes, ce complexe permet d'éliminer, diminuer ou inhiber la synthèse des radicaux libres de type ERN et/ou ERO, dont la synthèse en continu pourrait chroniciser la plaie.

De plus, le complexe formé par les colloïdes Col-2, avantageusement les colloïdes Col-2 de Pt, liés de manière covalente ou non covalente avec l'Aphanothece Sacrum Polysaccharides a un rôle antibactérien.

La matrice bipolymérique tridimensionnelle selon l'invention pour son utilisation comme pansement est plus efficace et plus rémanente pour prévenir et/ou cicatriser une plaie cutanée, notamment une radiodermite.

La matrice bipolymérique tridimensionnelle pour son utilisation comme pansement selon l'invention est également plus économique en raison de changements moins nombreux et moins traumatisant pour la lésion traitée et de la limitation de l'arrachement des nouvelles cellules épithéliales en formation.

De plus, la matrice bipolymérique tridimensionnelle pour son utilisation comme pansement selon l'invention offre une nouvelle alternative à l'hospitalisation des patients, dont l'évolution de la cicatrisation est désormais susceptible d'être supervisée à distance par les moyens classiques de télémédecine et de télésurveillance.

En effet et de manière innovante, lorsque la plaie est infectée, la prolifération de la population bactérienne conduit à une variation du pH, de préférence une augmentation du pH. Il s'ensuit un changement du niveau de valence du cérium. Il en résulte un changement de couleur de la matrice qui passe du transparent à une couleur, par exemple l'orange, pour indiquer la nécessité de changer le pansement et/ou de traiter l'infection de la plaie afin d'assurer une bonne cicatrisation et réduire les risques de complications pour le sujet (septicémie...).

Par ailleurs, la structure transparente de la matrice bipolymérique selon l'invention est susceptible de permettre à un praticien médical d'observer facilement, éventuellement à distance, l'évolution de la cicatrisation et d'identifier en temps réel toutes complications éventuelles.

La matrice bipolymérique tridimensionnelle et le pansement selon l'invention combinent plusieurs caractéristiques et fonctionnalités :

- être non toxique et non allergène ;
- être capable de protéger efficacement la plaie de l'environnement extérieur ;
- être capable de prévenir les risques d'infection ;
- être capable d'absorber les excès d'exsudats et de sang ;
- créer ou maintenir un environnement humide autour de la plaie ;
- accélérer et améliorer la cicatrisation ;
- maintenir une bonne isolation thermique autour de la plaie pour améliorer le flux sanguin et la migration des cellules épidermiques ;
- être respirant et autoriser les échanges gazeux entre la plaie et son environnement ;
- maintenir le pH à un niveau favorisant la croissance épithéliale tout en limitant la prolifération d'organismes dans la plaie ;
- pouvoir être retiré facilement sans adhérer à la plaie ;
- offrir une durée d'utilisation prolongée, c'est-à-dire être plus économique et moins traumatisant pour la plaie ;
- avoir une propriété absorbante intermédiaire entre les pansements hydrogel et hydrocolloïdes ;
- être protecteur ;
- être antioxydant ; et
- être antibactérien.

Selon un autre aspect, l'invention concerne un procédé de production d'une matrice bipolymérique tridimensionnelle telle que définie précédemment mettant en oeuvre deux solutions :

- une solution (A) comprenant des colloïdes Col-1 liés de manière non covalente avec un polysaccharide non sulfaté, une source de métaux bivalents, de préférence un métal alcalino-terreux, et des colloïdes Col-2 liés en surface et de manière non covalente avec un polymère biocompatible ; et
- une solution (B) comprenant un acidifiant et un polysaccharide sulfaté.

Selon un mode de réalisation privilégié, l'invention concerne un procédé de production d'une matrice bipolymérique tridimensionnelle telle que définie précédemment mettant en oeuvre deux solutions :

- une solution (A) comprenant des colloïdes Col-1 liés de manière non covalente avec un polysaccharide non sulfaté, une source de métaux bivalents, de préférence un métal alcalino-terreux, et des colloïdes Col-2 liés en surface et de manière covalente avec un agent apte à charger positivement le colloïde, avantageusement la cystéamine ; et
- une solution (B) comprenant un acidifiant et un polysaccharide sulfaté.

La matrice bipolymérique selon l'invention est obtenue par mélange des solutions (A) et (B).

Avantageusement, les deux solutions (A) et (B) sont mélangées dans une proportion de 4/5 de solution (A) et 1/5 de solution (B).

Selon un mode de réalisation particulier, dans les solutions (A) et (B) mises en oeuvre dans le procédé selon l'invention :

- les colloïdes Col-1 sont des colloïdes de dioxyde de cérium ;
- le polysaccharide non sulfaté est de l'alginate ;
- la source de métaux bivalents, de préférence un métal alcalino-terreux, est un sel de calcium, avantageusement le carbonate de calcium ;

- les colloïdes Col-2 sont des colloïdes de platine de degré d'oxydation zéro, avantageusement de platine dont le coeur est de degré d'oxydation zéro ;
- le polymère biocompatible est la polyvinylpyrrolidone ;
- l'acidifiant est l'acide gluconique ou la gluconolactone, avantageusement la gluconolactone ; et
- le polysaccharide sulfaté correspond au composé répondant à la désignation INCI Aphanothece Sacrum Polysaccharides modifié par ajout de groupements thiols.

Selon un autre mode de réalisation particulier, dans les solutions (A) et (B) mises en oeuvre dans le procédé selon l'invention :

- les colloïdes Col-1 sont des colloïdes de dioxyde de cérium ;
- le polysaccharide non sulfaté est de l'alginate ;
- la source de métaux bivalents, de préférence un métal alcalino-terreux, est un sel de calcium, avantageusement le carbonate de calcium ;
- les colloïdes Col-2 sont des colloïdes de platine de degré d'oxydation zéro, avantageusement de platine dont le coeur est de degré d'oxydation zéro, liés en surface et de manière covalente avec de la cystéamine ;
- l'acidifiant est la gluconolactone ; et
- le polysaccharide sulfaté correspond au composé répondant à la désignation INCI Aphanothece Sacrum Polysaccharides.

Selon l'invention, après le mélange des deux solutions (A) et (B), les réseaux polymériques sont réticulés et s'enchevêtrent, sans interaction mutuelle, formant une matrice bipolymérique tridimensionnelle sous forme de gel, avec un effet plastifiant, qui recouvre la lésion à traiter et maintenant autour de la plaie un film protecteur et respirant.

Avantageusement, la matrice bipolymérique selon l'invention correspond à un gel réticulé par des liaisons ioniques.

Selon un autre aspect, l'invention concerne un dispositif pour la mise en oeuvre du procédé selon l'invention.

Selon un mode de réalisation particulier, les deux solutions (A) et (B) sont conditionnées dans un flacon, un pulvérisateur, une seringue ou une unidose.

Selon un autre mode de réalisation particulier, les deux solutions (A) et (B) sont conditionnées dans un contenant bicompartimenté.

Selon un mode de réalisation particulier, le dispositif selon l'invention est caractérisé en ce que :

- la solution (A) est à pH 7, et comprend des colloïdes Col-1 liés de manière non covalente avec un polysaccharide non sulfaté, une source de métaux bivalents, de préférence un métal alcalino-terreux, et des colloïdes Col-2 liés de manière covalente avec un polymère biocompatible ; et
- la solution (B) est à pH 3, et comprend un acidifiant et un polysaccharide sulfaté.

Selon un autre mode de réalisation particulier, le dispositif selon l'invention est caractérisé en ce que :

- la solution (A) est à pH 7, et comprend des colloïdes Col-1 liés de manière non covalente avec un polysaccharide non sulfaté, une source de métaux bivalents, de préférence un métal alcalino-terreux, et des colloïdes Col-2 liés de manière covalente avec de la cystéamine ; et
- la solution (B) est à pH 3, et comprend un acidifiant et un polysaccharide sulfaté.

Selon un mode de réalisation particulier, dans les solutions (A) et (B) mises en oeuvre dans le dispositif selon l'invention :

- les colloïdes Col-1 sont des colloïdes de dioxyde de cérium ;
- le polysaccharide non sulfaté est de l'alginate ;
- la source de métaux bivalents, de préférence un métal alcalino-terreux, est un sel de calcium, avantageusement le carbonate de calcium ;
- les colloïdes Col-2 sont des colloïdes de platine de degré d'oxydation zéro, avantageusement de platine dont le coeur est de degré d'oxydation zéro ;
- le polymère biocompatible est la polyvinylpyrrolidone ;
- l'acidifiant est l'acide gluconique ou la gluconolactone, avantageusement la gluconolactone ; et
- le polysaccharide sulfaté correspond au composé répondant à la désignation INCI Aphanothece Sacrum Polysaccharides , modifié par ajout de groupements thiols.

Des exemples de contenants bicompartimentés adaptés à la formation de la matrice selon l'invention sont :

- la seringue bicompartimentée vendue par la société DOSEUROPE et consistant en une seringue double de 42 ml de volume spécifiquement conçue pour ratios 4 :1 (code produit : D-KART-050-04), du mélangeur statique L212 (code produit : M-50-212) et d'un pistolet seringue double 4 :1 (code produit : PIST-50-4 :1) ; ou
- . le tube bicompartimenté EASYMIX TUBE commercialisé par la société NEOPAC.

Selon un mode de réalisation préféré, dans les solutions (A) et (B) mises en oeuvre dans le dispositif selon l'invention :

- les colloïdes Col-1 sont des colloïdes de dioxyde de cérium ;
- le polysaccharide non sulfaté est de l'alginate ;
- la source de métaux bivalents, de préférence un métal alcalino-terreux, est un sel de calcium, avantageusement le carbonate de calcium ;
- les colloïdes Col-2 sont des colloïdes de platine de degré d'oxydation zéro, avantageusement de platine dont le coeur est de degré d'oxydation zéro, liés de manière covalente avec de la cystéamine ;
- l'acidifiant est la gluconolactone ; et
- le polysaccharide sulfaté correspond au composé répondant à la désignation INCI Aphanothece Sacrum Polysaccharides.

L'invention et les avantages qui en découlent ressortiront mieux des figures et des exemples suivants donnés afin d'illustrer l'invention et non de manière limitative.

[Fig. 1] La figure 1 représente le diffractogramme des colloïdes de $CeO_2$ avant liaison de manière non covalente avec de l'alginate.
[Fig. 2] La figure 2 représente le diffractogramme des colloïdes de Pt avant liaison de manière covalente avec de l'Aphanothece sacrum Polysaccharides (INCI), modifié par ajout de groupements thiols.
[Fig. 3] La figure 3 représente l'activité de dégradation de l'ABTS (en %) de la matrice bipolymérique selon l'invention par rapport à 2 produits commerciaux, en fonction du temps (en minutes).

EXEMPLES DE REALISATION DE L'INVENTION

1/ Synthèse des colloïdes de dioxyde de cérium (Col-1) selon l'invention

0,1-2mmol de dextrose ou de myo-inositol et 0,5-5 équivalents d'un précurseur de dioxyde de cérium tel que le chlorure de cérium sont solubilisés dans 10-100mL d'eau. A dissolution, 1-10 équivalents d'ammoniaque sont ajoutés. La solution est maintenue sous agitation pendant 1-5h. De l'acétone est ajouté en tant que contre solvant pour permettre une purification par centrifugation. Le culot est redispersé dans l'eau à la concentration voulue.
Le résultat de ce procédé de synthèse est des colloïdes de dioxyde de cérium enrobés de dextrose ou myo-inositol. Dans ce complexe, le dextrose et le myo-inositol assurent un rôle de contrôle de la taille des particules et de la dispersion étroite de la taille des particules autour d'une taille moyenne.

2/ Synthèse des colloïdes de platine (Col-2) selon l'invention, pour liaison covalente avec un polysaccharide sulfaté

Une solution est préparée à partir de 0,1 à 10 mmol de gluconolactone dissous dans 10 à 100 mL d'eau puis chauffée à reflux.
Quand le reflux est atteint, 0,01 à 10 mmol d'un sel précurseur de platine colloïdal tel que l'acide chloroplatinique et 0,001 à 1 mmol de polyvinylpyrrolidone (PVP) dissous dans 10 à 100 mL d'eau sont ajoutés au milieu réactionnel.
La solution est laissée à reflux pendant 1 à 5h.
La solution est ensuite refroidie à température ambiante et de l'acétone est ajouté en tant que contre solvant pour permettre une purification par centrifugation. Le culot est alors dispersé à la concentration voulue dans l'eau.
Le résultat de ce procédé de synthèse est des colloïdes de platine dispersés dans du PVP. Ces colloïdes peuvent être liés de façon covalente à un polysaccharide sulfaté selon l'invention, par exemple le l'Aphanothece Sacrum Polysaccharides (INCI), modifié par ajout de groupements thiols, dont la hémisynthèse est décrite dans l'exemple 3.

3/ Hémi-synthèse de l'Aphanothece Sacrum Polysaccharides (INCI), modifié par ajout de groupements thiols

10 à 100 x 10-7 mmol d'Aphanothece Sacrum Polysaccharides sont solubilisés dans 10 à100 mL d'eau et la solution est mise sous agitation.

100000-10000000 x 10-7 mmol de N-(3-Dimethylaminopropyl)-N'-ethylcarbodiimide hydrochloride sont ajoutés. La solution est laissée sous agitation pendant 10 à 100 min.

1000000 à 100000000 x 10-7 mmol de cystéine sont ajoutés, la solution est laissée sous agitation jusqu'à dissolution du solide. Le pH est ajusté à 4.

La solution est laissée sous agitation pendant 6 à 24h. Le pH est ensuite ajusté à 6.

La solution est versée dans environ dix fois son volume d'éthanol afin de faire précipiter l'Aphanothece Sacrum Polysaccharides modifié par ajout de groupements thiols et récupéré ce composé par centrifugation avant de le solubiliser dans un minimum d'eau (solution à 1-5% massique) pour être lyophilisé.

### 4/ Synthèse des colloïdes de platine (Col-2) selon l'invention, pour liaison non covalente avec le polysaccharide sulfaté

0,1-10mmol de gluconolactone sont dissouts dans 10-100mL d'eau et la solution est chauffée à reflux.

Quand le reflux est atteint, 0,1-1 équivalent d'un sel précurseur de platine colloïdal tel que l'acide chloroplatinique et 0,01-0,1 équivalent de polyvinylpyrrolidone (PVP) dissouts dans 10-100mL d'eau sont ajoutés au milieu réactionnel.

La solution est laissée à reflux pendant 1-5h. La solution est refroidie à température ambiante et 0,1-1 équivalent de chlorhydrate de cystéamine sont ajoutés. La solution est maintenue sous agitation pendant 1-5h puis de l'acétone est ajouté en tant que contre solvant pour permettre une purification par centrifugation. Le culot est alors dispersé à la concentration voulue dans l'eau.

Le résultat de ce procédé de synthèse est des colloïdes de platine modifié par ajout d'une cystéamine et dispersés dans du PVP. Ces colloïdes peuvent être liée de façon non-covalente (interaction coulombienne) à un polysaccharide sulfate, par exemple l'Aphanothece Sacrum Polysaccharides (INCI).

### 5/ Colloïdes $CeO_2$/alginate et Pt/Aphanothece Sacrum Polysaccharides selon l'invention

La petite taille des particules de $CeO_2$ et de Pt permet de disposer d'une aire plus importante à couvrir et donc de lier un plus grand nombre de molécules à la surface de chacune des particules.

Les diffractogrammes ont été mesurés sur poudre avec un DRX de source Cu-K$\alpha$ en transmission.

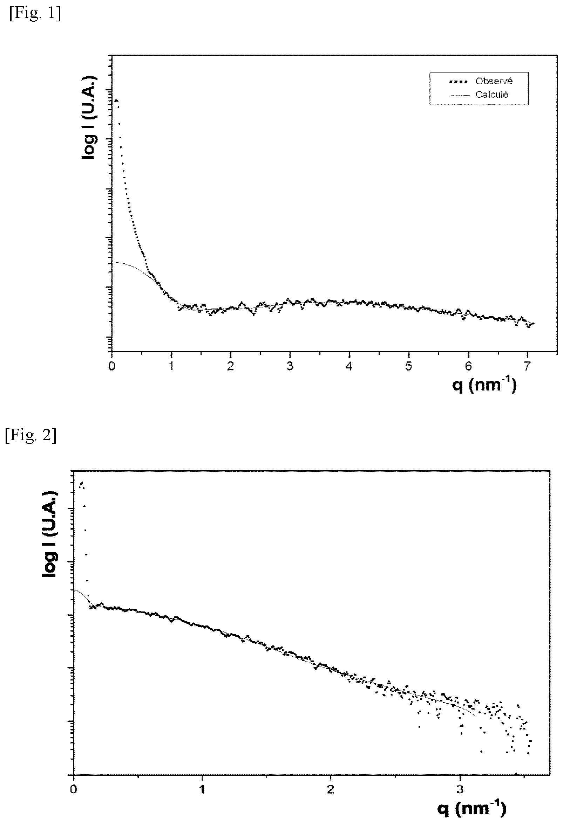

Le diffractogramme des colloïdes de $CeO_2$ avant liaison non covalente avec l'alginate est représenté par la figure 1.

Le diffractogramme des colloïdes de Pt avant liaison covalente avec l'Aphanothece sacrum Polysaccharides modifié par ajout de groupements thiols est représenté par la figure 2.

### 6/ Procédé de fabrication de la matrice bipolymérique selon l'invention

Deux solutions (A) et (B) sont mélangées dans une proportion de 4/5 de solution (A) et 1/5 de solution (B).

La solution (A) comprend des colloïdes $CeO_2$ selon l'invention issus de l'exemple 1 et de l'alginate, du carbonate de calcium, et des colloïdes Pt/Aphanothece Sacrum Polysaccharides selon l'invention issus du mélange des réactions décrites dans les exemples 2 et 3, et de l'eau ;

La solution (B) comprend l'Aphanothece Sacrum Polysaccharides (INCI) natif (non modifié) et de la gluconolactone dispersés dans l'eau.

Alternativement, la solution A comprend des colloïdes CeO2 selon l'invention issus de l'exemple 1, de l'alginate, du carbonate de calcium, des colloïdes de platine selon l'exemple 4 et de l'Aphanothece Sacrum Polysaccharides (INCI) natif (non modifié) et de l'eau ; et la solution B de l'Aphanothece Sacrum Polysaccharides natif (non modifié) et de la gluconolactone dispersés dans l'eau.

Après mélange, la matrice bipolymérique tridimensionnelle selon l'invention se forme en quelques minutes (1-5 minutes), voire secondes (moins d'une minute) par enchevêtrement des 2 réseaux polymériques réticulés selon l'invention.

### 7/ Détermination de l'activité antioxydante de la matrice bipolymérique tridimensionnelle selon l'invention

La matrice bipolymérique selon l'invention comprend :

- une quantité de colloïdes Col-1 liés de manière non covalente avec de l'alginate qui représente entre 0,01 et 1 % en poids de la matrice ;
- une quantité de colloïdes Col-2 liés de manière covalente avec l'Aphanothece sacrum Polysaccharides modifié par ajout de groupements thiols qui représente entre 0,001 et 0,1 % en poids de la matrice ;
- une quantité d'eau qui représente entre 70 et 99 % en poids de la matrice.

Une solution stock d'ABTS (acide 2,2'-azino-bis(3-éthylbenzothiazoline-6-sulphonique)) à 14 mM est incubée, à volume égal, avec une solution à 4,9 mM d'une solution hydraulique de persulfate d'ammonium pour produire un radical ABTS cationique. Le mélange réactionnel est incubé à l'obscurité pendant 16 heures à température ambiante. La solution obtenue est diluée au 1/100ème avec du tampon phosphate (0,2 M, et pH 7,4) contenant 150 mM de NaCl pour obtenir une absorbance de 1,5 à 734 nm.

Des échantillons de différentes masses comprises entre 1,2 et 12 mg de la matrice bipolymérique tridimensionnelle selon l'invention sont dispersés à 240 g/L dans l'eau sont ajoutés à 2970 μL de la solution cationique d'ABTS 0,07 mM dans l'eau, puis placés sous agitation dans le noir.

Après 30 minutes d'incubation, l'ABTS est totalement dégradé.

Dans une cuve de spectrophotométrie ultraviolet (UV) en polyméthacrylate de méthyle, le produit est mélangé à 3000 μL de la solution d'ABTS telle que préparée précédemment. Le mélange réactionnel est incubé à l'obscurité pendant 30 minutes à température ambiante. Ensuite, l'absorbance est mesurée toutes les 20 minutes.

La propriété antioxydante de la matrice bipolymérique selon l'invention est comparée à deux produits commerciaux :

- le pansement Nu-Gel® correspondant à un hydrogel contenant de l'alginate de sodium pour le traitement des plaies sèches, fibrineuses ou nécrotiques ;
- le pansement Flaminal Hydro® correspondant à un gel comprenant de l'alginate et un système enzymatique antimicrobien.

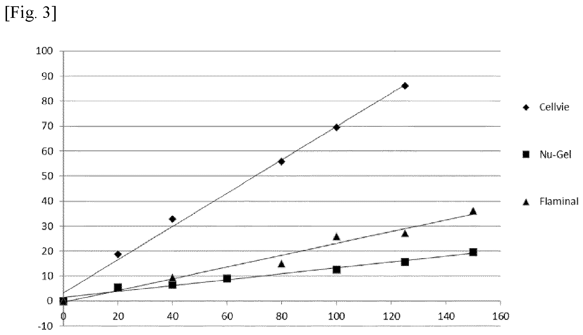

Les données sont représentées par la figure 3.

Les résultats montrent qu'après 120 heures, environ 85% d'ABTS ont été dégradé par la matrice bipolymérique selon l'invention alors que les pansements Nu-Gel® et Flaminal Hydro® dégradent respectivement environ 18% et 27% d'ABTS.

En conclusion, l'activité antioxydante de la solution contenant la matrice bipolymérique selon l'invention est exponentielle et largement supérieure comparée à celles des 2 pansements commerciaux.

8/ Détermination de la capacité d'absorption des exsudats par la matrice bipolymérique tridimensionnelle selon l'invention

La capacité d'absorption des exsudats par la matrice bipolymérique selon l'invention a été comparée à différents produits commerciaux indiqués pour le traitement de plaies exsudatives voire très exsudatives.

Une solution d'exsudat est préparée en mélangeant 50 mL de sérum de voeu foetal et 50 mL d'un diluant contenant 0,1 % de peptone et 0,9 % de chlorure de sodium. Les pansements en plaques sont découpés de façon à obtenir une surface de 1,98 cm$^2$. Les pansements en gel sont versés de façon à recouvrir une surface de 1,98 cm$^2$.

Chaque échantillon est placé dans une boite de Pétri de 3 cm de diamètre, puis pesé (m0). Chaque pansement est recouvert de 4 mL d'exsudat avant de sceller les boites de Pétri et de les conserver à 21°C pendant 72 heures. L'exsudat restant est retiré de chaque boite et chaque échantillon est à nouveau pesé (m1).

La capacité d'absorption des exsudats de chaque pansement est déterminée selon la formule suivante :

[Math 1]

$$\text{Capacité d'absorption} = \frac{m1 - m0}{m0} \times 100$$

Les résultats sont représentés dans le tableau 1 ci-dessous.

[Tableau 1]

| Marque de pansement | Permafoam Comfort | Biatain Adhesive | Intrasite Conformable | Hydrotac | Tegarderm Hydrocolloid Thin | Duoderm Extra Thin | Matrice bipolymérique selon l'invention |
|---|---|---|---|---|---|---|---|
| Type | Hydrofibre | Hydrocellulaire | Hydrogel en plaques | Hydrogel en plaques | Hydrocolloïde | Hydrocolloïde | Hydrogel en plaques |
| Absorption après 60h (g/ 100 cm²) | 66,25 | 101,60 | 11,64 | 35,41 | 19,81 | 30,90 | 15,36 |

Les résultats montrent que les pansements de types hydrofibre et hydrocellulaire, indiqués pour les plaies exsudatives voire très exsudatives, ont une meilleure absorption avec 65 à 100 g/cm$^2$ d'exsudat absorbé.

Les pansements de type hydrogel en plaques absorbent 10 à 35 g/cm$^2$ d'exsudat.

Les pansements de type hydrocolloïdes, indiqués pour le traitement des plaies peu exsudatives, absorbent entre 20 et 30 g/cm$^2$ d'exsudat.

La matrice bipolymérique selon l'invention absorbe 15 g/cm$^2$ d'exsudat, la situant dans les pansements de type hydrogel en plaques. Il s'agit du seul hydrogel en plaques sous forme liquide ayant supporté le test d'absorption des exsudats, puisque les 2 hydrogels commerciaux évalués (Intrasite Conformable et Hydrotac) se sont partiellement ou totalement dissous dans l'exsudat.

Ceci s'explique par la réticulation de la matrice bipolymérique selon l'invention qui permet d'absorber l'exsudat tout en conservant la forme du pansement.

De plus, l'absorption d'exsudat se fait lentement, graduellement, permettant un réarrangement de la matrice bipolymérique. Au contraire, les produits commerciaux testés absorbent l'exsudat comme une éponge. En conséquence, après saturation, ces produits ne peuvent plus absorber d'exsudat et ne sont donc plus efficaces.

**Revendications**

1. Matrice bipolymérique tridimensionnelle déployant une activité biomécanique, apte à neutraliser les différents paramètres physiopathologiques impliqués dans le développement et l'aggravation des lésions et/ou plaies cutanées combinant:

   - un premier réseau polymérique comprenant des premiers colloïdes (Col-1) liés de manière non covalente avec un polysaccharide réticulé non sulfaté; et
   - un second réseau polymérique réticulé comprenant des seconds colloïdes (Col-2) liés de manière covalente ou non covalente avec un polysaccharide sulfaté.

2. Matrice selon la revendication 1, **caractérisée en ce que** les colloïdes Col-1 sont des colloïdes de dioxyde de cérium et le polysaccharide non sulfaté est choisi dans le groupe comprenant : l'alginate, l'acide hyaluronique, la gomme guar, la gomme xanthane, la gomme acacia, le pullulan, le dextrane et leurs mélanges, avantageusement l'alginate.

3. Matrice selon l'une des revendications 1 à 2, **caractérisée en ce que** les colloïdes Col-2 sont des colloïdes de platine et le polysaccharide sulfaté est choisi dans le groupe comprenant le composé dont la désignation INCI est Aphanothece Sacrum Polysaccharides, les glycosaminoglycanes sulfatés, par exemple la dermatane sulfate, l'héparine, le sulfate d'héparane ou encore la chondroïtine sulfate ; les glucanes, les fucanes, les fucoïdanes, les carraghénanes, les ulvanes, le pentosane polysulfate et leurs mélanges, avantageusement l'Aphanothece Sacrum Polysaccharides.

4. Matrice selon l'une des revendications 1 à 3, **caractérisée en ce que** les seconds colloïdes (Col-2) sont liés de manière non covalente avec le polysaccharide sulfaté.

5. Matrice selon l'une des revendications 1 à 4, pour utilisation comme pansement pour prévenir et/ou cicatriser une plaie cutanée.

6. Matrice pour utilisation selon la revendication 5, **caractérisée en ce que** la plaie est sèche ou exsudative.

7. Matrice selon l'une des revendications 1 à 4, pour utilisation comme pansement pour prévenir et/ou cicatriser une radiodermite.

8. Matrice polymérique pour utilisation selon la revendication 7, **caractérisée en ce que** la radiodermite est de grade sec à exsudatif.

9. Procédé de production d'une matrice bipolymérique tridimensionnelle selon l'une des revendications 1 à 4, **caractérisé en ce que** la matrice est obtenue par le mélange :

   - une solution (A) comprenant des colloïdes Col-1 liés de manière non covalente avec un polysaccharide non sulfaté, une source de métaux bivalents, de préférence un métal alcalino-terreux, et des colloïdes Col-2 liés en surface et de manière covalente avec un agent apte à charger positivement le colloïde, avantageusement la cystéamine ; et
- d'une solution (B) comprenant un acidifiant et un polysaccharide sulfaté.

**10.** Procédé selon la revendication 9, **caractérisé en ce que** :

- les colloïdes Col-1 sont des colloïdes de dioxyde de cérium ;
- le polysaccharide non sulfaté est de l'alginate ;
- la source de métaux bivalents, de préférence un métal alcalino-terreux, est un sel de calcium, avantageusement le carbonate de calcium ;
- les colloïdes Col-2 sont des colloïdes de platine de degré d'oxydation zéro, avantageusement de platine dont le coeur est de degré d'oxydation zéro, liés en surface et de manière covalente avec un agent apte à charger positivement le colloïde, avantageusement la cystéamine;
- l'acidifiant est la gluconolactone ; et
- le polysaccharide sulfaté correspond au composé répondant à la désignation INCI Aphanothece Sacrum Polysaccharides.

**11.** Procédé selon l'une des revendications 9 à 10, **caractérisé en ce que** les deux solutions (A) et (B) sont mélangées dans une proportion de 4/5 de solution (A) et 1/5 de solution (B).

**12.** Dispositif pour la mise en oeuvre du procédé selon l'une des revendications 9 à 11 **caractérisé en ce que** les deux solutions sont conditionnées dans un flacon, un pulvérisateur, une seringue ou une unidose.

**13.** Dispositif selon la revendication 12, **caractérisé en ce que** les deux solutions sont conditionnées dans un contenant bicompartimenté.

**14.** Dispositif selon l'une des revendications 12 à 13, **caractérisé en ce que** :
la solution (A) est à pH 7, et comprend des colloïdes Col-1 liés de manière non covalente avec un polysaccharide non sulfaté, une source de métaux bivalents, de préférence un métal alcalino-terreux, et des colloïdes Col-2 liés de manière covalente avec un agent apte à charger positivement le colloïde, avantageusement la cystéamine ; et

- la solution (B) est à pH 3, et comprend un acidifiant et un polysaccharide sulfaté.

**15.** Dispositif selon l'une des revendications 12 à 14, **caractérisé en ce que** :
les colloïdes Col-1 sont des colloïdes de dioxyde de cérium ;

- le polysaccharide non sulfaté est de l'alginate ;
- la source de métaux bivalents, de préférence un métal alcalino-terreux, est un sel de calcium, avantageusement le carbonate de calcium ;
- les colloïdes Col-2 sont des colloïdes de platine de degré d'oxydation zéro, avantageusement de platine dont le coeur est de degré d'oxydation zéro, liés de manière covalente avec un agent apte à charger positivement le colloïde, avantageusement la cystéamine ;
- l'acidifiant est la gluconolactone;
- le polysaccharide sulfaté correspond au composé répondant à la désignation INCI Aphanothece Sacrum Polysaccharides.

**Patentansprüche**

**1.** Dreidimensionale, bipolymere Matrix mit biologischer und biomechanischer Aktivität, die in der Lage ist, die verschiedenen physiopathologischen Parameter zu neutralisieren, die bei der Entwicklung und Verschlechterung von Hautläsionen und/ oder Wunden eine Rolle spielen, indem sie Folgendes kombiniert:

- ein erstes Polymernetzwerk, das erste Kolloide (Col-1) umfasst, die nichtkovalent an ein nicht sulfatiertes, vernetztes Polysaccharid gebunden sind; und
- ein zweites vernetztes Polymernetzwerk, das zweite Kolloide (Col-2) umfasst, die kovalent oder nichtkovalent an ein sulfatiertes Polysaccharid gebunden sind.

**EP 3 999 132 B1**

2. Matrix nach Anspruch 1, **dadurch gekennzeichnet, dass** es sich bei den Kolloiden Col-1 um Kolloide aus Cerdioxid handelt und das nicht sulfatierte Polysaccharid ausgewählt wird aus der Gruppe zu der gehören: Alginat, Hyaluronsäure, Guargummi, Xanthangummi, Gummi arabicum, Pullulan, Dextran und ihre Mischungen, am besten Alginat.

3. Matrix nach einem der Ansprüche 1 bis 2, **dadurch gekennzeichnet, dass** es sich bei den Kolloiden Col-2 um Kolloide aus Platin handelt und das sulfatierte Polysaccharid, ausgewählt wird aus der Gruppe zu der gehören: die Verbindung mit der INCI - Bezeichnung Aphanothece Sacrum Polysaccharides, sulfatierte Glycosaminoglycane, zum Beispiel Dermatansulfat, Heparin, Heparansulfat oder auch Chondroitinsulfat; die Glucane, die Fucane, die Fucoidane, die Carraghenane, die Ulvane, Pentosanpolysulfat und ihre Mischungen, am besten Aphanothece Sacrum Polysaccharides.

4. Matrix nach einem der Ansprüche 1 bis 3, **dadurch gekennzeichnet, dass** die zweiten Kolloide (Col-2) nicht kovalent mit dem sulfatierten Polysaccharid verbunden sind.

5. Matrix nach einem der Ansprüche 1 bis 4, zur Verwendung als Verband zur Vermeidung und/ oder Vernarbung einer Hautverletzung.

6. Matrix zur Verwendung nach Anspruch 5, **dadurch gekennzeichnet, dass** die Wunde trocken oder feucht ist.

7. Matrix nach einem der Ansprüche 1 bis 4, zur Verwendung als Verband zur Vermeidung und/ oder Vernarbung einer Radiodermitis.

8. Polymere Matrix, zur Verwendung nach Anspruch 7, **dadurch gekennzeichnet, dass** die Radiodermitis vom Grad trocken bis feucht ist.

9. Herstellungsverfahren einer bipolymeren, dreidimensionalen Matrix, nach einem der Ansprüche 1 bis 4, **dadurch gekennzeichnet, dass** die Matrix hergestellt wird durch die Mischung:

   - einer Lösung (A), die Kolloide Col-1 enthält, die in nicht kovalenter Weise an ein nicht sulfatiertes Polysaccharid gebunden sind, eine Quelle bivalenter Metalle, vorzugsweise ein Erdalkalimetal, sowie Kolloide Col-2, oberflächlich und kovalent an einen Wirkstoff gebunden, der in der Lage ist, das Kolloid positiv zu laden, vorteilhafterweise Cysteamin; und
   - einer Lösung (B) mit einem Säuerungsmittel und einem sulfatierten Polysaccharid.

10. Verfahren nach Anspruch 9, **dadurch gekennzeichnet, dass**:

    - es sich bei den Kolloiden Col-1 um Cerdioxid-Kolloide handelt;
    - es sich bei dem nicht sulfatierten Polysaccharid um Alginat handelt;
    - die Quelle bivalenter Metalle, vorzugsweise ein Erdalkalimetal, ein Kalziumsalz, vorteilhafterweise Kalziumkarbonat ist;
    - es sich bei den Kolloiden Col-2 um Platinkolloiden der Oxidationszahl Null handelt, vorteilhafterweise Platin, dessen Kern eine Oxidationszahl Null, aufweist, oberflächlich und kovalent an einen Wirkstoff gebunden, der in der Lage ist, das Kolloid positiv zu laden, vorteilhafterweise Cysteamin;
    - es sich beim Säuerungsmittel um Gluconolacton handelt; und
    - das sulfatierte Polysaccharid der Verbindung mit der INCI - Bezeichnung Aphanothece Sacrum entspricht.

11. Verfahren nach einem der Ansprüche 9 bis 10 **dadurch gekennzeichnet, dass** die beiden Lösungen (A) und (B) in einem Verhältnis von 4/5 Lösung (A) und 1/5 Lösung (B) vermischt werden.

12. Vorrichtung zur Umsetzung des Verfahrens nach einem der Ansprüche 9 bis 11, **dadurch gekennzeichnet, dass** die beiden Lösungen in einem Flakon, einem Zerstäuber, einer Spritze oder einer Einzeldosis abgefüllt werden.

13. Vorrichtung nach Anspruch 12, **dadurch gekennzeichnet, dass** die beiden Lösungen in einem zweigeteilten Behälter abgefüllt sind.

14. Vorrichtung nach einem der Ansprüche 12 bis 13, **dadurch gekennzeichnet, dass**: die Lösung (A) einen pH-Wert von 7 hat, und Kolloide Col-1 enthält, die in nicht kovalenter Weise an ein nicht sulfatiertes Polysaccharid gebunden sind, eine Quelle bivalenter Metalle, vorzugsweise ein Erdalkalimetal, sowie Kolloide Col-2, oberflächlich und ko-

17

valent an einen Wirkstoff gebunden, der in der Lage ist, das Kolloid positiv zu laden, vorteilhafterweise Cysteamin; und

- eine Lösung (B) einen pH-Wert von 3 hat, und ein Säuerungsmittel und ein sulfatiertes Polysaccharid enthält.

15. Vorrichtung nach einem der Ansprüche 12 bis 14, **dadurch gekennzeichnet, dass**: die Kolloide Col-1 Kolloide aus Cerdioxid sind:

- das nicht sulfatierte Polysaccharid ist Alginat;
- die Quelle bivalenter Metalle, vorzugsweise ein Erdalkalimetal, ist ein Kalziumsalz, vorteilhafterweise Kalziumkarbonat;
- bei den Kolloiden Col-2 handelt es sich um Platinkolloide der Oxidationszahl Null, vorteilhafterweise Platin, dessen Kern eine Oxidationszahl Null, aufweist, kovalent an einen Wirkstoff gebunden, der in der Lage ist, das Kolloid positiv zu laden, vorteilhafterweise Cysteamin;
- beim Säuerungsmittel handelt es sich um Gluconolacton;
- das sulfatierte Polysaccharid entspricht der Verbindung mit der INCI - Bezeichnung Aphanothece Sacrum.

## Claims

1. Three-dimensional bipolymeric matrix deploying biomechanical activity, capable of neutralizing the different physio-opathological parameters involved in the development and worsening of skin lesions and/or wounds combining:

   - a first polymeric network comprising first colloids (Col-1) non-covalently linked with a non-sulphated cross-linked polysaccharide; and
   - a second cross-linked polymeric network comprising second colloids (Col-2) covalently or non-covalently linked with a sulphated polysaccharide.

2. Matrix according to claim 1, **characterized in that** the Col-1 colloids are cerium dioxide colloids and the non-sulfated polysaccharide is chosen from the group comprising: alginate, hyaluronic acid, guar gum, xanthan gum, acacia gum, pullulan, dextran and their mixtures, advantageously alginate.

3. Matrix according to one of claims 1 to 2, **characterized in that** the Col-2 colloids are platinum colloids and the sulfated polysaccharide is chosen from the group comprising the compound whose INCI designation is Aphanothece Sacrum Polysaccharides, sulfated glycosaminoglycans, for example dermatan sulfate, heparin, heparan sulfate or even chondroitin sulfate; glucans, fucans, fucoidans, carrageenans, ulvans, pentosan polysulfate and their mixtures, advantageously Aphanothece Sacrum Polysaccharides.

4. Matrix according to one of claims 1 to 3, **characterized in that** the second colloids (Col-2) are non-covalently linked with the sulfated polysaccharide.

5. Matrix according to one of claims 1 to 4, for use as a dressing to prevent and/or heal a skin wound.

6. Matrix for use according to claim 5, **characterized in that** the wound is dry or exudative.

7. Matrix according to one of claims 1 to 4, for use as a dressing for prevent and/or heal radiodermatitis.

8. Polymeric matrix for use according to claim 7, **characterized in that** radiodermatitis is dry to exudative grade.

9. Method for producing a three-dimensional bipolymeric matrix according to one of claims 1 to 4, **characterized in that** the matrix is obtained by the mixture:

   - a solution (A) comprising Col-1 colloids non-covalently linked with a non-sulfated polysaccharide, a source of divalent metals, preferably an alkaline earth metal, and Col-2 colloids covalently linked on the surface with an agent capable of positively charging the colloid, advantageously cysteamine; and
   - a solution (B) comprising an acidifier and a sulfated polysaccharide.

10. Method according to claim 9, **characterized in that**:

- Col-1 colloids are cerium dioxide colloids;
- the non-sulfated polysaccharide is alginate;
- the source of divalent metals, preferably an alkaline earth metal, is a calcium salt, advantageously calcium carbonate;
- the Col-2 colloids are platinum colloids with a zero oxidation state, advantageously platinum whose core has a zero oxidation state, covalently linked on the surface with an agent capable of positively charging the colloid, advantageously cysteamine;
- the acidifier is gluconolactone; and
- the sulfated polysaccharide corresponds to the compound corresponding to the INCI designation Aphanothece Sacrum Polysaccharides.

11. Method according to one of claims 9 to 10, **characterized in that** the two solutions (A) and (B) are mixed in a proportion of 4/5 solution (A) and 1/5 solution (B).

12. Device for implementing the method according to one of claims 9 to 11, **characterized in that** the two solutions are packaged in a bottle, a sprayer, a syringe, or a single dose.

13. Device according to claim 12, **characterized in that** the two solutions are packaged in a two-compartment container.

14. Device according to one of claims 12 to 13, **characterized in that**:

- solution (A) is at pH 7, and comprises Col-1 colloids non-covalently linked with a non-sulfated polysaccharide, a source of divalent metals, preferably an alkaline earth metal, and Col-2 colloids covalently linked with an agent capable of positively charging the colloid, advantageously cysteamine; and
- solution (B) is at pH 3 and includes an acidifier and a sulfated polysaccharide.

15. Device according to one of claims 12 to 14, **characterized in that**:

- Col-1 colloids are cerium dioxide colloids;
- the non-sulfated polysaccharide is alginate;
- the source of divalent metals, preferably an alkaline earth metal, is a calcium salt, advantageously calcium carbonate;
- the Col-2 colloids are platinum colloids with a zero oxidation state, advantageously platinum whose core has a zero oxidation state, covalently linked with an agent capable of positively charging the colloid, advantageously cysteamine;
- the acidifier is gluconolactone;
- the sulfated polysaccharide corresponds to the compound corresponding to the INCI designation Aphanothece Sacrum Polysaccharides.

[Fig. 1]

[Fig. 2]

[Fig. 3]

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 3466454 A